(19) Europäisches Patentamt
European Patent Office
Office européen des brevets

(11) Veröffentlichungsnummer : **0 050 775**
**B1**

# (12) EUROPÄISCHE PATENTSCHRIFT

(45) Veröffentlichungstag der Patentschrift : **14.09.83**

(21) Anmeldenummer : **81108189.2**

(22) Anmeldetag : **12.10.81**

(51) Int. Cl.³ : **C 07 C 51/60**, C 07 C 63/00, B 01 J 31/24, C 08 G 63/40, C 08 G 69/32

## (54) Verfahren zur Herstellung von aromatischen Carbonsäurechloriden.

(30) Priorität : **25.10.80 DE 3040295**

(43) Veröffentlichungstag der Anmeldung : **05.05.82 Patentblatt 82/18**

(45) Bekanntmachung des Hinweises auf die Patenterteilung : **14.09.83 Patentblatt 83/37**

(84) Benannte Vertragsstaaten : **DE FR GB IT NL**

(56) Entgegenhaltungen :
**DE B 2 321 122**
**GB A 1 127 095**

(73) Patentinhaber : **BAYER AG**
**Zentralbereich Patente, Marken und Lizenzen**
**D-5090 Leverkusen 1, Bayerwerk (DE)**

(72) Erfinder : **Rudolph, Udo, Dr.**
**Scheiblerstrasse 101**
**D-4150 Krefeld (DE)**
Erfinder : **Freitag, Dieter, Dr.**
**Hasenheide 10**
**D-4150 Krefeld 1 (DE)**
Erfinder : **Bottenbruch, Ludwig, Dr.**
**Woehlerstrasse 5**
**D-4150 Krefeld 1 (DE)**
Erfinder : **Schmidt, Manfred, Dr.**
**Bodelschwinghstrasse 20**
**D-4150 Krefeld 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 050 775 B1

# Verfahren zur Herstellung von aromatischen Carbonsäurechloriden

Die Erfindung betrifft ein Einstufenverfahren zur Herstellung von hochreinen, polykondensationsfähigen aromatischen Carbonsäurechloriden mit Thionylchlorid und einem tert. Phosphinoxid bzw. dessen Umsetzungsprodukt mit Thionylchlorid und/oder dem sich bildenden Säurechlorid als Katalysator. Die Umsetzung von aliphatischen und aromatischen Carbonsäuren mit Thionylchlorid ist ein Standardverfahren in der organischen präparativen Chemie zur Herstellung der entsprechenden Säurechloride. Allerdings werden hierbei dunkelgefärbte Reaktionsprodukte erhalten, die nur einen Carbonsäurechloridgehalt von 96-99 Gew.-% aufweisen. Aromatische Dicarbonsäuredichloride dieses geringen Reinheitsgrades können z. B. nicht direkt nach dem Zweiphasengrenzflächenverfahren zur Herstellung von hochmolekularen Polykondensaten, wie aromatischen Polyamiden oder aromatischen Polyestern, umgesetzt werden. Ihr Gehalt an nicht oder nur halbseitig umgesetzten Dicarbonsäuren stört die Polykondensation, verursacht unerwünschten Kettenabbruch, und ergibt Polymere mit endständigen Carboxylgruppen und im Falle der Polyester Polymere mit zusätzlichen Anhydridstrukturen. Bei der Herstellung von Säurechloriden mit Thionylchlorid und Dimethylformamid als Katalysator (vergl. DE-PS 1 026 750) können als Nebenprodukt erhebliche Mengen von Dimethylcarbamidsäurechlorid entstehen, die das Produkt darüber hinaus zusätzlich verunreinigen oder sich im evtl. zurückgeführten überschüssigen Thionylchlorid akkumulieren.

Weiter enthalten so hergestellte Säurechloride Schwefel in elementarer und vor allem gebundener Form, der ebenfalls einen störenden Einfluß auf die Eigenschaften des resultierenden Polykondensates ausübt.

Die DE-B 23.21.122 beschreibt ein Verfahren zur Herstellung von Carbonsäurechloriden durch Umsetzung von Carbonsäuren mit Phosgen in Gegenwart von trisubstituierten Phosphinoxiden als Katalysator. Zwar liefert dieses Verfahren die Carbonsäurechloride in guten Ausbeuten und hoher Reinheit, hat aber den Nachteil, daß Phosgen als Chlorierungsmittel verwendet wird.

Um farblose Dicarbonsäuredichloride mit einer Reinheit ≥ 99,9 % nach diesem Verfahren zu erhalten, müssen die Produkte durch Umkristallisation oder Destillation gereinigt werden. Dies erfordert zusätzlichen Aufwand und vermindert die Ausbeute ; bei der Destillation besteht die Gefahr einer spontanen Zersetzung.

Bei der Umsetzung von Thionylchlorid mit aromatischen Mono-, Di- und Tricarbonsäuren in Gegenwart eines tert. Phosphinoxids bzw. dessen Umsetzungsprodukt mit Thionylchlorid und/oder dem sich bildenden Säurechlorid als Katalysator erhält man farblose aromatische Carbonsäurechloride mit einem COOH-Gehalt ≤ 0,05 % und einem Gesamtschwefelgehalt ≤ 50 ppm, so daß sie ohne Nachreinigung zur Herstellung von farblosen, hochmolekularen Polykondensaten eingesetzt werden können.

Gegenstand der Erfindung ist also ein Einstufenverfahren zur Herstellung von reinen, insbesondere auch schwefelfreien aromatischen Carbonsäurechloriden durch Umsetzung der entsprechenden Säuren mit Thionylchlorid, das dadurch gekennzeichnet ist, daß man als Katalysatoren tert. Phosphinoxide bzw. deren Umsetzungsprodukte mit Thionylchlorid und/oder den sich bildenden Säurechloriden verwendet. Die erfindungsgemäß als Katalysatoren eingesetzten Phosphinoxide und deren Folgeprodukte können ohne nachteilige Wirkung auf die Eigenschaften der herzustellenden Polykondensats im Säurechlorid verbleiben.

Als erfindungsgemäß wirksame Katalysatoren sind tert. Phosphinoxide der allgemeinen Struktur (I) geeignet,

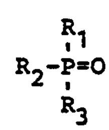

(I)

wobei $R_1$, $R_2$ und $R_3$ gleich oder verschieden, $C_1$-$C_8$-Alkyl, $C_6$-$C_{10}$-Aryl, $C_7$-$C_{20}$-Alkylaryl- oder Arylalkyl sein können. Bevorzugt sind $R_1$, $R_2$ und $R_3$ $C_6$-$C_{12}$-Arylreste, wie Phenyl oder mit $C_1$-$C_4$-Alkylresten substituiertes Phenyl. Die so näher bezeichneten Kohlenwasserstoffreste $R_1$, $R_2$ und $R_3$ können zusätzlich, z. B. durch Halogen substituiert sein.

Geeignete Katalysatoren sind :

Triisopropylphosphinoxid, Tributylphosphinoxid,
Trihexylphosphinoxid ; Triphenylphosphinoxid,
Tri-1-naphthylphosphinoxid, Tri-2-biphenylylphosphinoxid, Tri-4-biphenylphosphinoxid, Tris-4-methylphenyl-phosphinoxid, Diphenyl-(4-methylphenyl)-phosphinoxid, Phenyl-bis-4-methylphenyl-phosphinoxid ; Tribenzylphosphinoxid ; Dimethylbenzylphosphinoxid, Methyldiphenyl-phosphinoxid, Diethylphenylphosphinoxid, Ethylphenylbenzylphosphinoxid und Bis-chlormethyl-(3-chlorphenyl)-phosphinoxid.

Besonders geeignet ist Triphenylphosphinoxid.

Erfindungsgemäß werden 0,1 bis 5,0 Gew.-%, vorzugsweise 0,1 bis 1,0 Gewichtsprozent, bezogen auf

die eingesetzten aromatischen Säuren, an tert. Phosphinoxiden der allgemeinen Struktur (I) eingesetzt.

Im Prinzip können alle aromatischen Carbonsäuren eingesetzt werden. Sie entsprechen insbesondere den folgenden Formeln (II) bis (VII) :

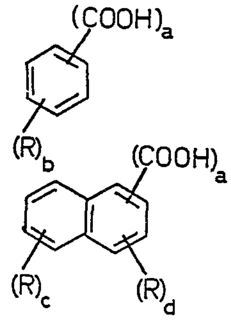

(II)

(III)

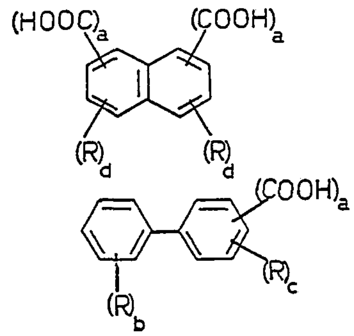

(IV)

(V)

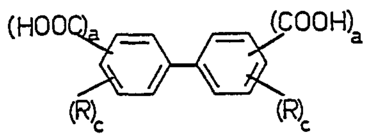

(VI)

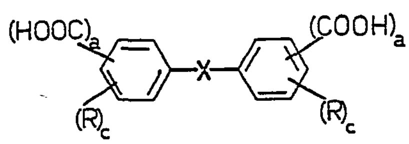

(VII)

wobei R ein Substituent aus der Gruppe von Alkylgruppen mit ein bis sechs Kohlenstoffatomen, halogensubstituierten Alkylgruppen mit ein bis sechs Kohlenstoffatomen, Alkoxygruppen und ebenfalls den entsprechenden halogensubstituierten Alkoxygruppen mit ein bis sechs Kohlenstoffatomen und Halogenatomen sein kann,

X steht für einen Ethersauerstoff, eine Methylengruppe oder Isopropylengruppe, einen $C_5$-$C_7$-Cycloalkylenrest oder eine —C = O—Gruppe, während a eine ganze Zahl von 1 bis 3 (im allgemeinen sind nicht mehr als drei COOH-Gruppen pro Molekül vorhanden), b eine ganze Zahl von 0 bis 5, c eine ganze Zahl von 0 bis 4, und d eine ganze Zahl von 0 bis 3 repräsentiert.

Beispielhaft seien genannt :

Phthalsäure, Isophthalsäure, Terephthalsäure, Gemische aus Iso- und Terephthalsäure, 4,4'-Dicarboxybenzophenon, Diphensäure, 1,4-Naphthalindicarbonsäure und Trimesinsäure.

Zur Durchführung des erfindungsgemäßen Verfahrens können die aromatischen Carbonsäuren nach Zugabe der erfindungsgemäßen Katalysatoren mit 1-2 mol Thionylchlorid pro Carboxylgruppe versetzt und diese Suspension oder Lösung auf 50-150 °C, vorzugsweise 80-100 °C, gebracht werden.

Nach Abdestillieren des überschüssigen Thionylchlorids und kurzzeitigem Anlegen von Vakuum bei Reaktionstemperatur erhält man einen Rückstand, der zu ≥ 99,9 Gew.-% aus aromatischem Carbonsäurechlorid besteht.

Es ist möglich, das Verfahren diskontinuierlich oder kontinuierlich durchzuführen.

In den Beispielen sind Prozentangaben auf Gewicht bezogen.

## Beispiel 1

In einem 1-l-Dreihalskolben mit Rührer, Thermometer und Rückflußkühler werden vorgelegt und aufgeheizt :

83 g Isophthalsäure
83 g Terephthalsäure
357 g Thionylchlorid
0,83 g Triphenylphosphinoxid

Die farblose Suspension wird innerhalb von 30 min auf 60-80 °C aufgeheizt, wobei zügige HCl- und $SO_2$-Entwicklung auftritt. Nach ca. 8 h ist die Gasentwicklung beendet. Es liegt eine hellgelbe Lösung vor, aus der das überschüssige Thionylchlorid bei Normaldruck und danach im Vakuum bei 100 °C entfernt wird.

Der farblose Rückstand enthält neben dem Katalysator :

≥ 99,9 % Säurechlorid
≤ 0,05 % COOH
≤ 5 ppm Gesamtschwefel
≤ 0,05 % $Cl^-$.

Ausbeute : 100 %

Beispiel 2

210 g Trimesinsäure,
267,7 g Thionylchlorid und
1,05 g Triphenylphosphinoxid

liefern in der gleichen Weise wie in Beispiel 1 beschrieben Trimesinsäuretrichlorid. Es enthält neben dem Katalysator

≥ 99,9 % Säurechlorid
≤ 0,05 % COOH
≤ 50 ppm Gesamtschwefel
≤ 0,05 % $Cl^-$.

Ausbeute : 100 %

Beispiel 3

166 g Isophthalsäure,
261,8 g Thionylchlorid und
1 g Tributylphosphinoxid

ergeben in der gleichen Weise wie in Beispiel 1 beschrieben Isophthalsäuredichlorid. Es enthält neben dem Katalysator

≥ 99,9 % Säurechlorid
≤ 0,05 % COOH
≤ 5 ppm Gesamtschwefel
≤ 0,05 % $Cl^-$.

Ausbeute : 100 %

Beispiele 4-10

166 g Isophthalsäure und
261,8 g Thionylchlorid

wurden nacheinander in Gegenwart von je 1 g Trisiopropylphosphinoxid (4), Trihexylphosphinoxid (5), Dimethylbenzylphosphinoxid (6), Tribenzylphosphinoxid (7), Methyldiphenylphosphinoxid (8), Ethylphenylbenzylphosphinoxid (9) und Bis-(chlormethyl-(3-chlorphenyl)-phosphinoxid umgesetzt.

Die Produkte hatten alle die gleichen analytischen Kennzahlen und Ausbeuten wie das in Beispiel 3 beschriebene.

**Ansprüche**

1. Einstufenverfahren zur Herstellung von reinen, insbesondere auch schwefelfreien aromatischen

Carbonsäurechloriden durch Umsetzung einer aromatischen Carbonsäure mit ein bis drei Carboxylgruppen oder eines entsprechenden aromatischen Carbonsäuregemisches mit Thionylchlorid in Anwesenheit eines Katalysators, dadurch gekennzeichnet, daß man als Katalysatoren tert. Phosphinoxide bzw. deren Folgeprodukte mit Thionylchlorid und/oder dem zu bildenden Säurechlorid einsetzt.

2. Verfahren nach Anspruch 1, dadurch gekennzeichnet, daß das eingesetzte tert. Phosphinoxid eine Verbindung der Formel I

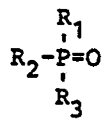

(I)

ist, in der

$R_1$, $R_2$ und $R_3$ gleich oder verschieden sind und $C_1$-$C_8$-Alkyl, $C_6$-$C_{12}$-Aryl, $C_7$-$C_{20}$-Alkylaryl oder Arylalkyl bedeuten.

3. Verwendung der gemäß Anspruch 1 erhaltenen aromatischen Dicarbonsäuredi- und Tricarbonsäuretrichloride zur Herstellung von Polykondensaten.

**Claims**

1. A single-stage process for the production of pure, particularly also sulphur-free, aromatic carboxylic acid chlorides by reacting an aromatic carboxylic acid containing one to three carboxyl groups or a corresponding mixture of aromatic carboxylic acids with thionyl chloride in the presence of a catalyst, characterised in that tertiary phosphine oxides or their reaction products with thionyl chloride and/or the acid chloride to be formed are used as catalysts.

2. Process according to Claim 1, characterised in that the tertiary phosphine oxide used is a compound of the formula I

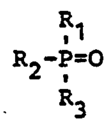

(I)

in which

$R_1$, $R_2$ and $R_3$ are identical or different and denote $C_1$-$C_8$-alkyl, $C_6$-$C_{12}$-aryl, $C_7$-$C_{20}$-alkylaryl or arylalkyl.

3. Use of the aromatic dicarboxylic acid dichlorides and tricarboxylic acid trichlorides obtained according to Claim 1 for the production of polycondensates.

**Revendications**

1. Procédé à une seule étape pour la production de chlorure d'acides carboxyliques aromatiques purs, en particulier également dépourvus de soufre, par réaction d'un acide carboxylique aromatique porteur d'un à trois groupes carboxyle ou d'un mélange d'acides carboxyliques aromatiques correspondants avec le chlorure de thionyle en présence d'un catalyseur, caractérisé en ce qu'on utilise comme catalyseurs des oxydes de phosphines tertiaires ou leurs dérivés, avec le chlorure de thionyle et/ou le chlorure d'acide correspondant.

2. Procédé suivant la revendication 1, caractérisé en ce que l'oxyde de phosphine tertiaire utilisé est un composé de formule I

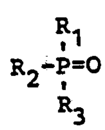

(I)

dans laquelle

$R_1$, $R_2$ et $R_3$ sont égaux ou différents et désignent des groupes alkyle en $C_1$ à $C_8$, aryle en $C_6$ à $C_{12}$, alkylaryle ou arylalkyle en $C_7$ à $C_{20}$.

3. Utilisation des dichlorures d'acides dicarboxyliques et trichlorures d'acides tricarboxyliques aromatiques obtenus suivant la revendication 1 pour l'obtention de produits de polycondensation.